# EUROPEAN PATENT SPECIFICATION

(11) **EP 3 822 610 B1**
(45) Date of publication and mention of the grant of the patent: **04.12.2024**
(21) Application number: 19833574.7
(22) Date of filing: 02.07.2019
(51) Int. Cl.: G01N 1/10, G01M 3/04, G01M 3/06, G01N 1/22, G01N 33/00

(54) **CLAMP FOR DISSOLVED-GAS MONITOR**
KLEMME FÜR MONITOR VON AUFGELÖSTEM GAS
PINCE POUR DÉTECTEUR DE GAZ DISSOUS

(30) Priority: 12.07.2018 JP 2018132059
(43) Date of publication of application: 19.05.2021
(73) Proprietor: Pureron Japan Co., Ltd., Iwaki-shi, Fukushima 970-1144 (JP)
(72) Inventor: NAKAJIMA, Hidetoshi, Iwaki-shi, Fukushima 970-1144 (JP); ABE, Yasuhiro, Iwaki-shi, Fukushima 973-8408 (JP); TAGUCHI, Koji, Iwaki-shi, Fukushima 973-8408 (JP)
(74) Representative: Schön, Christoph
(86) International application number: PCT/JP2019/026383
(87) International publication number: WO 2020/013040

(56) References cited:
- EP-A1- 3 312 584
- CA-A1- 2 613 883
- JP-A- 2011 017 575
- JP-B1- 5 859 159
- JP-U- S5 084 091
- US-A1- 2014 311 220

## Description

### Technical Field

The present invention relates to a clamp for dissolved-gas monitor device which measures the concentration of gas dissolved in an aqueous solution of the gas circulating through the inside of a pipe which allows the permeation of a specific gas.

### Background Art

Conventionally, a dissolved-hydrogen monitor device is known which measures the hydrogen concentration of hydrogen water circulating through the inside of a pipe, such as a tube, which allows the permeation of hydrogen in non-contact with the hydrogen water (for example, see Patent Document 1).

In the device of Patent Document 1, a tubular component is used as a jacket member which is formed to surround the outer periphery of the pipe and forms a sealed space for storing hydrogen gas, and therefore the airtightness of the sealed space can be easily secured. Thus, the hydrogen concentration of the hydrogen water circulating through the inside of the pipe can be securely measured by the use of the device.
CA 2 613 883 A1 discloses a clamp for dissolved-gas monitor which measures a concentration of gas dissolved in an aqueous solution of the gas flowing through an inside of a pipe which allows permeation of a specific gas. The clamp for dissolved-gas monitor according to document D1 comprises a chamber for collecting gas in the bottom of the probe insert formed by side walls and an upper wall, wherein the bottom of the side walls is sealingly engaged to the outer surface of the pipe to be monitored using compressible sealing means.

### Citation List

### Patent Literature

Patent Literature 1: Japanese Patent No. 5859159

### Summary of Invention

### Technical Problem

However, a tubular component cannot be used as a jacket member which forms a sealed space for storing gas permeating through a pipe in some cases, e.g., a case where a dissolved-gas monitor device is installed in order to measure the concentration of gas dissolved in an aqueous solution of the gas circulating through the inside of an existing pipe.

In such a case, it is considered to use components which sandwich the outer periphery of the pipe from the outside, such as a clamp, as the jacket member which forms the sealed space. However, in order to secure the airtightness of the bonded surface of the components, high machining accuracy has been demanded, which has posed a problem of an increase in the manufacturing cost of the components.

In view of the problem of the conventional technique, it is an object of the present invention to provide a clamp for dissolved-gas monitor capable of securing the airtightness of a sealed space for storing gas permeating through a pipe with a simple configuration.

### Solution to Problem

A clamp for dissolved-gas monitor according to the present invention which is defined in appended claim 1 measures the concentration of gas dissolved in an aqueous solution of the gas circulating through the inside of a pipe which allows the permeation of a specific gas and includes a pair of pipe pressing portions which sandwiches the pipe and a fixing portion which detachably fixes the pair of pipe pressing portions such that the pair of pipe pressing portions sandwiches the pipe, in which one of the pair of pipe pressing portions has a gas storage portion which is a space which is opened to the side of the pipe and stores gas permeating through the pipe and an exhaust port which exhausts the gas stored in the gas storage portion.

The clamp for dissolved-gas monitor of the present invention includes the pair of pipe pressing portions which sandwiches the pipe, such as a tube, and includes the gas storage portion which is the space which is opened to the side of the pipe and stores the gas permeating through the pipe in one of the pipe pressing portions.

In the state where the pair of pipe pressing portions is fixed to sandwich the pipe, such as a tube, a space surrounded by a part of the outer peripheral surface of the pipe and the gas storage portion forms a sealed space which stores the gas permeating through the pipe.

Thus, a portion where the airtightness needs to be secured is only a portion where the pipe and the pipe pressing portion including the gas storage portion abut on each other. The pipe, such as a tube, has elasticity, and thus can be easily stuck to the pipe pressing portions. Therefore, the airtightness of the sealed space formed by the outer peripheral surface of the pipe and the gas storage portion can be secured with a simple configuration.

Further, one of the pair of pipe pressing portions has the exhaust port which exhausts the gas stored in the gas storage portion. By arranging a sensor which detects the gas such that the airtightness is maintained at the tip of the exhaust port, the gas permeating through the pipe passes through the exhaust port to reach the sensor, so that the concentration of the gas is measured.

As described above, the present invention can provide the clamp for dissolved-gas monitor capable of securing the airtightness of the sealed space for storing the gas permeating through the pipe with a simple configuration.

In the clamp for dissolved-gas monitor according to the present invention, the pipe pressing portion having the gas storage portion of the pair of pipe pressing portions has a re-permeable membrane which allows the permeation of the gas permeating through the pipe and the re-permeable membrane is arranged to cover the entire surface of an opening portion of the gas storage portion.

The clamp for dissolved-gas monitor of the present invention has the re-permeable membrane which allows the permeation of the gas permeating through the pipe. The re-permeable membrane is arranged to cover the entire surface of the opening portion of the gas storage portion.

Since the clamp for dissolved-gas monitor is freely attachable/detachable, it is considered to repeatedly install and remove the clamp for dissolved-gas monitor on/from the pipe which is a gas concentration measurement target. When the clamp is repeatedly installed on and removed from the pipe, there is a risk that foreign substances are mixed into the gas storage portion.

According to the present invention, the airtightness of the gas storage portion is more easily secured with the re-permeable membrane arranged to cover the entire surface of the opening portion of the gas storage portion and, even when the clamp is repeatedly installed on and removed from the pipe, foreign substances can be prevented from mixing into the gas storage portion and a failure of the sensor accompanying the mixing of the foreign substances can be prevented, and therefore the concentration of the gas can be more securely measured.

As described above, the present invention can provide the clamp for dissolved-gas monitor capable of more securely measuring the concentration of the gas while more easily securing the airtightness of the sealed space for storing the gas permeating through the pipe.

In the clamp for dissolved-gas monitor according to the present invention, it is preferable that the re-permeable membrane is arranged to cover the entire surface of the opening portion of the gas storage portion and the entire surface on the side of the pipe of the pipe pressing portion having the gas storage portion.

The clamp for dissolved-gas monitor of the present invention is arranged such that the re-permeable membrane covers the entire surface of the opening portion of the gas storage portion and the entire surface on the side of the pipe of the pipe pressing portion having the gas storage portion.

Thus, the gas permeates to the gas storage portion from a portion covering the opening portion but the gas does not permeate from a portion other than the portion covering the opening portion because the portion is closed by the surface on the side of the pipe of the pipe pressing portion having the gas storage portion.

Therefore, the leakage of the gas can be prevented, and therefore the concentration of the gas can be more accurately measured.

As described above, the present invention can provide the clamp for dissolved-gas monitor capable of more accurately measuring the concentration of the gas while preventing the leakage of the gas.

The clamp for dissolved-gas monitor according to the present invention preferably has a support portion which supports a part of the outer peripheral surface of the pipe between the surface of the gas storage portion and the pipe.

The clamp for dissolved-gas monitor of the present invention has the support portion between the surface of the gas storage portion and the pipe. The support portion supports a part of the outer peripheral surface of the pipe.

The re-permeable membrane allows the permeation of the gas permeating through the pipe and the re-permeable membrane is arranged to cover the entire surface of the opening portion of the gas storage portion. The support portion may support the outer peripheral surface of the pipe through the re-permeable membrane.

When the pipe expands to the side of the gas storage portion due to water pressure or the like of the aqueous solution of the gas flowing through the inside of the pipe or contracts after the expansion, the capacity of the gas storage portion varies, so that there is a risk that a concentration measurement environment cannot be kept constant.

The present invention can prevent the expansion of the pipe to the side of the gas storage portion due to water pressure or the like of the aqueous solution of the gas flowing through the inside of the pipe. Therefore, a reduction in the capacity of the gas storage portion due to the expansion of the pipe can also be prevented, and therefore the concentration of the gas can be measured while keeping the concentration measurement environment constant.

As described above, the clamp for dissolved-gas monitor can be provided which can measure the concentration of the gas while preventing the pipe from expanding to the side of the gas storage portion and keeping the gas concentration measurement environment constant.

### Brief Description of Drawings

FIG. 1 is a reference view illustrating an example of the entire configuration of a clamp for dissolved-gas monitor of this embodiment.
FIG. 2 is a cross-sectional view of a use state of the clamp for dissolved-gas monitor of this embodiment.
FIG. 3 is a cross-sectional view of the use state of the clamp for dissolved-gas monitor of this embodiment.
FIG. 4 is a schematic view of the use state of the clamp for dissolved-gas monitor of this embodiment.

### Description of Embodiments

Next, an embodiment for carrying out the present invention is described in more detail with reference to the attached drawings. The same configurations are designated by the same reference numerals and descriptions thereof are omitted in some cases.

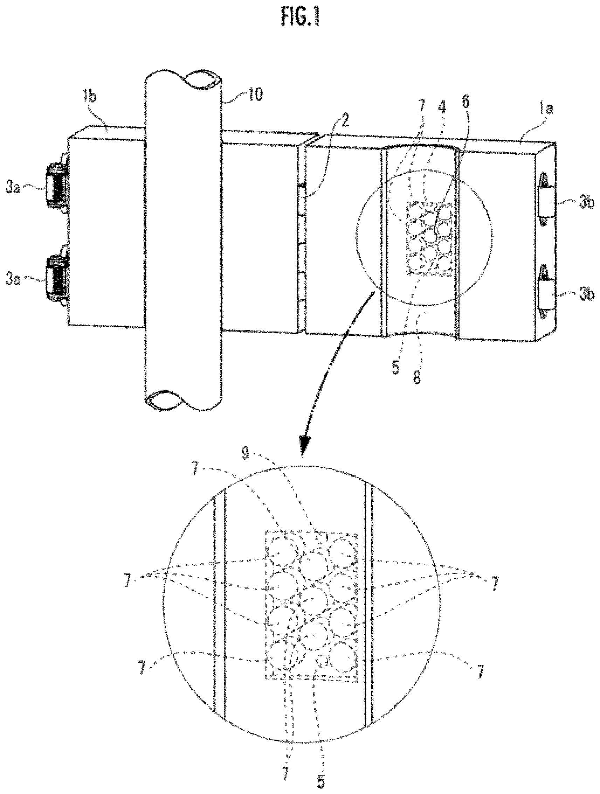

First, the configuration of a clamp for dissolved-gas monitor of this embodiment is described with reference to FIG. 1.

The clamp for dissolved-gas monitor includes a pair of pipe pressing portions 1 sandwiching a pipe 10, a hinge 2, and fixing portions 3.

The pair of pipe pressing portions 1 contains a first pipe pressing portion 1a and a second pipe pressing portion 1b. The first pipe pressing portion 1a has a gas storage portion 4, an exhaust port 5, and an air supply port 9. The second pipe pressing portion 1b does not have the gas storage portion 4.

The hinge 2 is fixed to one side surfaces of the first pipe pressing portion 1a and the second pipe pressing portion 1b and rotatably supports the first pipe pressing portion 1a and the second pipe pressing portion 1b.

The fixing portions 3 are configured to detachably fix the first pipe pressing portion 1a and the second pipe pressing portion 1b such that the first pipe pressing portion 1a and the second pipe pressing portion 1b sandwich the pipe 10 and are catch clips each containing a locking portion 3a and a lock receiving portion 3b, for example. The fixing portions 3 are fixed to the side surfaces of the first pipe pressing portion 1a and the second pipe pressing portion 1b opposite to the side surfaces to which the hinge 2 is fixed. The number of the fixing portions 3 may be one or two or more.

The gas storage portion 4 is a space which stores gas permeating through the pipe 10 and is opened to the side of the pipe 10.

The exhaust port 5 is a hole which exhausts the gas stored in the gas storage portion 4. One side is opened to the surface of the gas storage portion 4 and the other side penetrates through the first pipe pressing portion 1a and is opened to the opposite surface of the first pipe pressing portion 1a, for example.

In the state where the first pipe pressing portion 1a and the second pipe pressing portion 1b are rotated with the hinge 2 as the fulcrum to sandwich the pipe 10, such as a tube, and are fixed using the fixing portions 3, a space surrounded by a part of the outer peripheral surface of the pipe 10 and the gas storage portion 4 forms a sealed space which stores gas permeating through the pipe 10. More specifically, gas permeating through from the entire periphery of the pipe 10 is not stored and gas permeating through from a part of the pipe 10 is stored.

Thus, a portion where airtightness needs to be secured is only a portion where the pipe 10 and the first pipe pressing portion 1a including the gas storage portion 4 abut on each other. The pipe 10, such as a tube, has elasticity and is easily stuck to the first pipe pressing portion 1a. Therefore, the airtightness of the sealed space formed by the outer peripheral surface of the pipe 10 and the gas storage portion 4 can be secured with a simple configuration.

Further, the first pipe pressing portion 1a has the exhaust port 5 which exhausts the gas stored in the gas storage portion 4. Therefore, by arranging a sensor capable of detecting the gas is arranged at the tip of the exhaust port 5 such that the airtightness is maintained, the gas permeating through the pipe 10 passes through the exhaust port 5 to reach the sensor, so that the concentration of the gas is measured.

As described above, the present invention can provide the clamp for dissolved-gas monitor capable of securing the airtightness of the sealed space for storing the gas permeating through the pipe 10 with a simple configuration.

Further, the first pipe pressing portion 1a has a re-permeable membrane 6 which allows the permeation of the gas permeating through the pipe 10. The re-permeable membrane 6 is a membrane formed of a material which allows the permeation of gas and is arranged to cover the entire surface of the opening portion of the gas storage portion 4. More specifically, even in a state where the fixation by the fixing portions 3 is released, a structure where the sealed space is not released is set.

Thus, the airtightness of the gas storage portion 4 can be more easily secured and, even when the pipe 10 is repeatedly installed on and removed from the clamp, foreign substances can be prevented from mixing into the gas storage portion 4 and a failure of the sensor accompanying the mixing of the foreign substances can be prevented, and therefore the concentration of the gas can be more securely measured.

As described above, the present invention can provide the clamp for dissolved-gas monitor capable of more securely measuring the concentration of the gas while more easily securing the airtightness of the sealed space for storing the gas permeating through the pipe 10.

Alternatively, the re-permeable membrane 6 may also be arranged to cover the entire surface of the opening portion of the gas storage portion 4 and the entire surface on the side of the pipe 10 of the pipe pressing portion 1 having the gas storage portion 4 (i.e., first pipe pressing portion 1a).

Thus, the gas permeates to the gas storage portion 4 from a portion covering the opening portion but the gas does not permeate from a portion other than the portion covering the opening portion because the portion is closed by the surface on the side of the pipe 10 of the pipe pressing portion 1 having the gas storage portion 4.

Therefore, the leakage of the gas can be prevented, and therefore the concentration of the gas can be more accurately measured.

As described above, the present invention can provide the clamp for dissolved-gas monitor capable of more accurately measuring the concentration of the gas while preventing the leakage of the gas.

Further, the first pipe pressing portion 1a has one or more support portions 7 which support the outer peripheral surface of the pipe 10 between the surface of the gas storage portion 4 and the pipe 10.

This makes it possible to prevent the pipe 10 from expanding to the side of the gas storage portion 4 due to water pressure or the like of an aqueous solution of the gas flowing through the inside of the pipe 10. Therefore, a reduction in the capacity of the gas storage portion 4 due to the expansion of the pipe 10 can also be prevented. Hence, the concentration of the gas can be measured while keeping the concentration measurement environment constant.

As described above, the present invention can provide the clamp for dissolved-gas monitor capable of measuring the concentration of the gas while keeping the gas concentration measurement environment constant by preventing the pipe 10 from expanding to the side of the gas storage portion 4.

The air supply port 9 is a hole for supplying, to the gas storage portion 4, the other gas for exhausting the gas stored in the gas storage portion 4 to the outside of the gas storage portion 4 through the exhaust port 5. One side of the air supply port 9 is opened to the surface of the gas storage portion 4 and the other side penetrates through the first pipe pressing portion 1a and is opened to the opposite surface of the first pipe pressing portion 1a, for example.

As the other gas for exhausting the gas permeating through the pipe 10 and stored in the gas storage portion 4 to the outside of the gas storage portion 4 through the exhaust port 5, gas is used which is substantially inert to the gas permeating through the pipe 10 and stored in the gas storage portion 4.

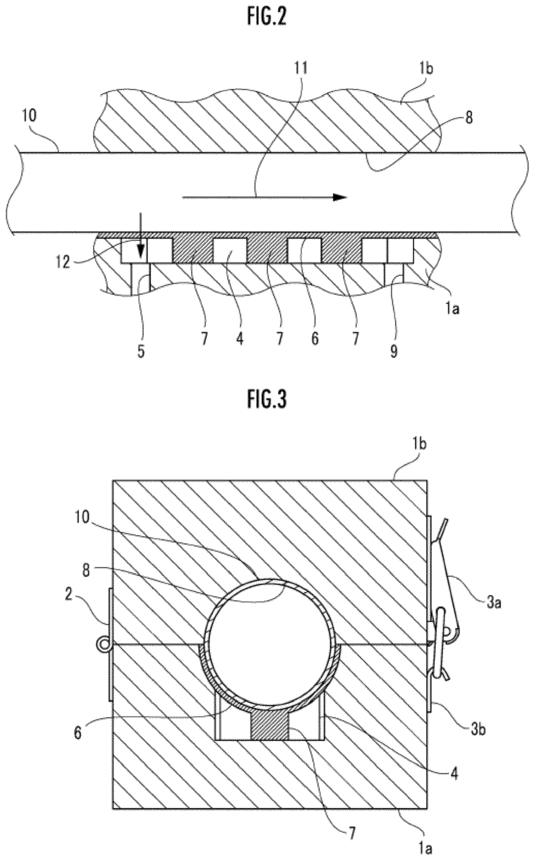

Next, an example of the use state of the clamp for dissolved-gas monitor of this embodiment is described using FIG. 2 to FIG. 4.

FIG. 2 is a cross-sectional view in which the use state of the clamp for dissolved-gas monitor of this embodiment is viewed from the side. FIG. 3 is a cross-sectional view in which the use state of the clamp for dissolved-gas monitor of this embodiment is viewed from the front.

In FIG. 2, the first pipe pressing portion 1a and the second pipe pressing portion 1b are fixed by the fixing portions 3 in the state of sandwiching the pipe 10.

The aqueous solution of the gas flows through the inside of the pipe 10 in a direction indicated by an arrow 11. Then, a part of the gas dissolved in the aqueous solution permeates through the surface on the side of the gas storage portion 4 of the pipe 10 and the re-permeable membrane 6, and then flows into the gas storage portion 4 in a course indicated by an arrow 12 and stored therein.

The gas stored in the gas storage portion 4 is exhausted through the exhaust port 5 together with the other gas substantially inert to the gas flowing into the gas storage portion 4 through the air supply port 9.

As illustrated in FIG. 3, the surface of the pair of pipe pressing portions 1 has a groove portion 8 with a semicircular cross portion. The groove portion 8 is formed such that the inner diameter of a cylindrical shape formed when the first pipe pressing portion 1a and the second pipe pressing portion 1b are fixed such that the first pipe pressing portion 1a and the second pipe pressing portion 1b sandwich the pipe 10 and the outer diameter of the pipe 10 are equal to each other, and therefore the pipe 10 can be inserted into the groove portion 8 such that the pipe 10 exactly closely contacts the groove portion 8.

As illustrated in FIG. 2 and FIG. 3, the support portions 7 support the outer peripheral surface of the pipe 10. In this example, 11 support portions 7 are integrally formed and arranged on the surface of the re-permeable membrane 6. The support portions 7 prevent the pipe 10 from expanding to the side of the gas storage portion 4 due to water pressure or the like of the aqueous solution of the gas flowing through the inside of the pipe 10. Further, these support portions 7 are arranged at appropriate intervals from one another, and therefore do not block the movement of the gas in the gas storage portion 4 and the other gas flowing in from the air supply port 9.

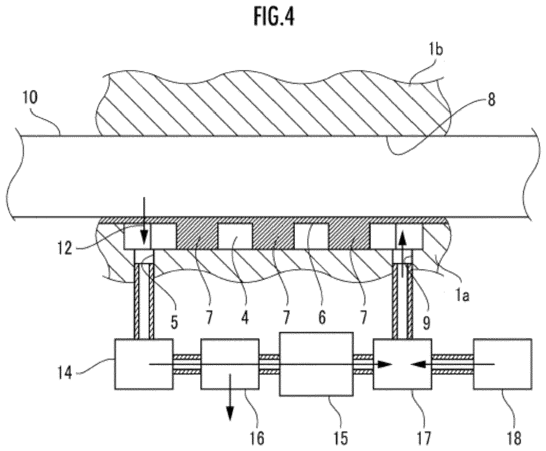

FIG. 4 is a schematic view of the use state of the clamp for dissolved-gas monitor of this embodiment.

By storing the gas permeating through the pipe 10 in the gas storage portion 4 and detecting the concentration of the gas in gas containing the gas (i.e., mixed gas of the gas permeating from the pipe 10 and the other gas flowing in from the air supply port 9) with a sensor 14, the concentration of the gas in the pipe 10 can be measured without being accompanied by contamination of the aqueous solution of the gas flowing through the inside of the pipe 10.

To make the concentration of the gas in the gas storage portion 4 follow a variation of the concentration of the gas in the pipe 10, the gas in the gas storage portion 4 is circulated for appropriate ventilation. For this ventilation, a circulation pump 15, an exhaust pump 16, and an air supply pump 17 are used as illustrated in FIG. 4.

Specifically, the mixed gas of the gas permeating from the pipe 10 and the other gas is drawn to the side of the exhaust port 5 by the circulation pump 15 to be guided to the sensor 14.

The mixed gas after the measurement of the gas concentration is further carried to the side of the air supply port 9 by the circulation pump 15. At this time, appropriate ventilation is performed by the exhaust pump 16 and the air supply pump 17 such that the concentration of the gas contained in the aqueous solution and the concentration of the gas of the mixed gas in the gas storage portion 4 reach the equilibrium state. To the air supply pump 17, gas substantially inert to the gas is supplied from a tank 18 as necessary.

Due to the fact that the mixed gas in the gas storage portion 4 is ventilated as described above, the concentration of the gas dissolved in the aqueous solution of the gas circulating through the inside of the pipe 10 and the concentration of a specific gas of the mixed gas in the gas storage portion 4 become equilibrium in due course and the subsequent equilibrium state is maintained.

Then, when the concentration of the gas dissolved in the aqueous solution of the gas circulating through the inside of the pipe 10 and the concentration of the specific gas of the mixed gas in the gas storage portion 4 has become equilibrium, the dissolved-gas monitor measures the concentration of the specific gas contained in the mixed gas by the sensor 14.

The timing when the concentration of the gas dissolved in the aqueous solution of the gas circulating through the inside of the pipe 10 and the concentration of the specific gas of the mixed gas in the gas storage portion 4 reach equilibrium can be found by the fact that the measured concentration becomes constant for a predetermined period of time by the measurement with time of the concentration of the specific gas of the mixed gas by the sensor 14.

As described above, the present invention can provide the clamp for dissolved-gas monitor capable of securing the airtightness of the sealed space for storing the gas permeating through the pipe with a simple configuration.

For example, Example describes the configuration of using the plurality of pumps in order to forcibly discharge the gas in the gas storage portion 4 to the outside of the gas storage portion 4 but the gas in the gas storage portion 4 may be discharged to the outside of the gas storage portion 4 by natural ventilation. In this case, the clamp for dissolved-gas monitor does not include the circulation pump 15, the exhaust pump 16, and the air supply pump 17 and may not necessarily include the air supply port 9.

### Description of Reference Numerals

- 1a: first pipe pressing portion
- 1b: second pipe pressing portion
- 2: hinge
- 3: fixing portion
- 4: gas storage portion
- 5: exhaust port
- 6: re-permeable membrane
- 7: support portion
- 8: groove portion
- 9: air supply port
- 10: pipe

## Claims

1. A clamp for dissolved-gas monitor which measures a concentration of gas dissolved in an aqueous solution of the gas flowing through an inside of a pipe (10) which allows permeation of a specific gas,
the clamp for dissolved-gas monitor comprising:
a pair of pipe pressing portions (1a, 1b) which sandwiches the pipe (10); and
a fixing portion (3) which detachably fixes the pair of pipe pressing portions (1a, 1b) such that the pair of pipe pressing portions (1a, 1b) sandwiches the pipe (10), wherein
one of the pair of pipe pressing portions (1a) has a gas storage portion (4) which is a space which is opened to a side of the pipe (10) and stores gas permeating through the pipe (10) and an exhaust port (5) which exhausts the gas stored in the gas storage portion (4), and
the pipe pressing portion (1a) having the gas storage portion (4) of the pair of pipe pressing portions (1a, 1b) has a re-permeable membrane (6) which allows permeation of the gas permeating through the pipe (10), **characterized in that**
the re-permeable membrane (6) is arranged so that a surface thereof on the pipe side contacts an outer circumference of the pipe (10) and so that an entire surface of an opening portion of the gas storage portion (4) is covered.

2. The clamp for dissolved-gas monitor according to Claim 1, wherein
the re-permeable membrane (6) is arranged to cover the entire surface of the opening portion of the gas storage portion (4) and an entire surface on a side of the pipe (10) of the pipe pressing portion (1a) having the gas storage portion (4).

3. The clamp for dissolved-gas monitor according to any one of Claims 1 to 2 comprising:
a support portion (7) which supports a part of an outer peripheral surface of the pipe (10) between a surface of the gas storage portion (4) and the pipe (10).

## Patentansprüche

1. Klemme für ein Überwachungsgerät für gelöste Gase, das eine Konzentration eines Gases misst, das in einer wässrigen Lösung des Gases gelöst ist, die durch das Innere eines Rohres (10) fließt, welches die Permeation eines bestimmten Gases ermöglicht, wobei die Klemme für das Überwachungsgerät für gelöste Gase Folgendes umfasst:
ein Paar von Rohrpressabschnitten (1a, 1b), das das Rohr (10) sandwichartig einklemmt, und
einen Fixierungsabschnitt (3), der das Paar von Rohrpressabschnitten (1a, 1b) lösbar fixiert, so dass das Paar von Rohrpressabschnitten (1a, 1b) das Rohr sandwichartig einklemmt,
wobei
ein Element (1a) des Paars von Rohrpressabschnitten einen Gasspeicherabschnitt (4), bei dem es sich um einen Raum handelt, der zu einer Seite des Rohrs (10) hin offen ist und Gas, das durch das Rohr (10) permeiert, speichert, sowie eine Auslassöffnung (5), die das in dem Gasspeicherabschnitt (4) gespeicherte Gas ablässt, aufweist, und
der Rohrpressabschnitt (1a) mit dem Gasspeicherabschnitt (4) des Paars von Rohrpressabschnitten (1a, 1b) eine re-permeabele Membran (6) aufweist, die eine Permeation des durch das Rohr (10) hindurch permeierenden Gases ermöglicht,
**dadurch gekennzeichnet, dass** die re-permeabele Membran (6) so angeordnet ist, dass eine Oberfläche hiervon auf der Rohrseite sich mit dem Außenumfang des Rohres (10) in Kontakt befindet und so angeordnet ist, dass eine gesamte Oberfläche eines Öffnungsabschnitts des Gasspeicherabschnitts (4) bedeckt ist.

2. Klemme für ein Überwachungsgerät für gelöste Gase gemäß Anspruch 1, wobei die re-permeabele Membran (6) so angeordnet ist, dass sie die gesamte Oberfläche des Öffnungsabschnitts des Gasspeicherabschnitts (4) und eine gesamte Oberfläche auf einer Seite des Rohrs (10) des Rohrpressabschnitts (1a), der den Gasspeicherabschnitt (4) aufweist, bedeckt.

3. Klemme für ein Überwachungsgerät für gelöste Gase gemäß einem der Ansprüche 1 bis 2, umfassend:
einen Stützabschnitt (7), der einen Teil einer äußeren peripheren Oberfläche des Rohrs (10) zwischen einer Oberfläche des Gasspeicherabschnitts (4) und dem Rohr (10) stützt.

## Revendications

1. Pince pour dispositif de surveillance de gaz dissous qui mesure une concentration de gaz dissous dans une solution aqueuse du gaz s'écoulant à travers l'intérieur d'un tuyau (10) qui permet la perméation d'un gaz spécifique, la pince pour dispositif de surveillance de gaz dissous comprenant :
une paire de sections de compression de tuyau (1a, 1b) qui prennent en sandwich le tuyau (10) ; et
une section de fixation (3) qui fixe de manière amovible la paire de sections de compression de tuyau (Ia, Ib) de sorte que la paire de sections de compression de tuyau (Ia, Ib) prenne en sandwich le tuyau (10),
dans laquelle l'une des deux sections de compression de tuyau (1a) comporte une section de stockage de gaz (4) qui est un espace qui est ouvert sur un côté du tuyau (10) et stocke le gaz pénétrant à travers le tuyau (10) et un orifice d'échappement (5) qui évacue le gaz stocké dans la section de stockage de gaz (4), et
la section de compression de tuyau (1a) comportant la section de stockage de gaz (4) de la paire de sections de compression de tuyau (1a, Ib) comporte une membrane reperméable (6) qui permet la perméation du gaz pénétrant à travers le tuyau (10),
**caractérisée en ce que** la membrane reperméable (6) est disposée de telle sorte qu'une de ses surfaces côté tuyau entre en contact avec une circonférence extérieure du tuyau (10) et de telle sorte qu'une surface entière d'une section d'ouverture de la section de stockage de gaz (4) soit recouverte.

2. Pince pour dispositif de surveillance de gaz dissous selon la revendication 1, dans laquelle la membrane perméable (6) est conçue pour recouvrir toute la surface de la section d'ouverture de la section de stockage de gaz (4) et toute une surface sur un côté du tuyau (10) de la section de compression de tuyau (1a) comportant la section de stockage de gaz (4).

3. Pince pour dispositif de surveillance de gaz dissous selon l'une quelconque des revendications 1 à 2, comprenant :
une section de support (7) qui supporte une partie d'une surface périphérique extérieure du tuyau (10) entre une surface de la section de stockage de gaz (4) et le tuyau (10).
